# EUROPÄISCHE PATENTSCHRIFT

(11) **EP 0 710 104 B1**
(45) Veröffentlichungstag und Bekanntmachung des Hinweises auf die Patenterteilung: **04.11.1998**
(21) Anmeldenummer: 94923715.0
(22) Anmeldetag: 08.07.1994
(51) Int. Cl.: A61K 9/48, A61K 47/14, A61K 47/26, A61K 47/44

(54) **PHARMAZEUTISCHE PRÄPARATE FÜR SCHWERLÖSLICHE WIRKSTOFFE**
PHARMACEUTICAL COMPOSITIONS FOR HARDLY SOLUBLE ACTIVE SUBSTANCES
PREPARATIONS PHARMACEUTIQUES POUR PRINCIPES ACTIFS DIFFICILEMENT SOLUBLES

(30) Priorität: 08.07.1993 DE 4322826
(43) Veröffentlichungstag der Anmeldung: 08.05.1996
(73) Patentinhaber: Novartis AG, 4058 Basel (CH)
(72) Erfinder: POSANSKI, Ulrich, D-79110 Freiburg (DE)
(86) Internationale Anmeldenummer: EP9402248
(87) Internationale Veröffentlichungsnummer: WO9501786

(56) Entgegenhaltungen:
- EP-A- 0 274 870
- WO-A-93/00891
- BE-A- 676 581
- GB-A- 2 228 198

## Beschreibung

Die vorliegende Erfindung betrifft pharmazeutische Präparate für schwerlösliche Wirkstoffe sowie Verfahren zur Herstellung dieser Präparate.

Generell bietet die Verabreichung eines pharmazeutischen Wirkstoffs durch orale Darreichungsformen, wie Tabletten, Kapseln oder Dragées, Vorteile gegenüber anderen, z.B. parenteralen Darreichungsformen. Rein subjektiv werden Krankheiten, die durch Injektionen behandelt werden müssen, als gravierender im Vergleich mit anderen Krankheiten empfunden, bei denen die Verabreichung von Tabletten, Kapseln oder Dragées kaum wahrgenommen wird. Besonders vorteilhaft ist die Eignung solcher Darreichungsformen zur Verabreichung durch den Patienten selbst, während parenterale Darreichungsformen, abgesehen von wenigen Ausnähmen, vom Arzt bzw. beauftragtem medizinischem Hilfspersonal appliziert werden müssen.

Nach Verabreichung und Zerfall einer oralen Darreichungsform wirkt die Flüssigkeit des Gastrointestinaltrakts, z.B. Magen- oder Darmsaft, auf den Wirkstoff ein. Viele oral zu applizierende Wirkstoffe haben lipophile Eigenschaften und sind daher im wässrigen Milieu des Gastrointestinaltrakts schlecht löslich. Es mindert sich dann die resorptionsfähige Menge des Wirkstoffs, so dass seine Bioverfügbarkeit abnimmt. Dies erfordert generell eine höhere Dosierung des zu applizierenden Wirkstoffes. Die Folge sind erhöhte biologische Variabilität und unerwünschte Schwankungen in der Wirksamkeit.

Zur Verbesserung der Löslichkeit sind für schwerlösliche Wirkstoffe sogenannte Löslichkeitsvermittler beschrieben, z.B. hydrophile Kosolvenzien wie Ethanol, Propylenglycol, flüssige Polyethylenglycole oder lipophile Löslichkeitsvermittler wie Lecithin, Fettsäurepolyglycolester oder Fettsäureglycerinpolyglycolester. Die Verwendung solcher Löslichkeitsvermittler ist wegen verminderter Verträglichkeit und mangelnder Stabilität der Darreichungsform, z.B. Entmischungseffekten, problematisch.

Man hat daher in der Deutschen Offenlegungsschrift 40 05 190 die Verwendung von Glycerinfettsäurepartialestern oder Partialestern von Propylenglycol vorgeschlagen. Diese Hilfsstoffe (Co-Tenside) sind nachteilig, da sie nur in dem engen HLB-Bereich von 2 bis 3 erhältlich sind. Dies erlaubt nur eine beschränkte Variation der Mengenverhältnisse der Bestandteile der Trägerzusammensetzung zwecks Anpassung an die unterschiedlichen Löslichkeiten der zu solubilisierenden Wirkstoffe.

Der vorliegenden Erfindung liegt die Aufgabe zugrunde, durch Auswahl besonders geeigneter Hilfsstoffe die Löslichkeit, Resorptionsfähigkeit und folglich auch die Bioverfügbarkeit von oral zu applizierenden Wirkstoffen zu erhöhen bzw. zu verbessern.

Diese Aufgabenstellung wird durch die vorliegende Erfindung gelöst, welche eine besonders vorteilhafte pharmazeutische Zusammensetzung zur verbesserten Solubilisierung eines in Wasser schwerlöslichen Wirkstoffs in der Trägerzusammensetzung betrifft Die Trägerzusammensetzung gemäss vorliegender Erfindung besteht aus folgenden Komponenten:
a) 10-50 Gew.-%, bezogen auf die Trägerzusammensetzung, eines im wesentlichen reinen, oder als Gemisch vorliegenden Co-Tensids, mit einem Hydrophil-Lipophil-Gleichgewicht Kleiner als 10 (HLB-Wert nach Griffin), ausgewählt aus der Gruppe Polyglycerinfettsäureester und Sorbitanfettsäureester;
b) 5-40 Gew.-%, bezogen auf die Trägerzusammensetzung, eines im wesentlichen reinen, oder als Gemisch vorliegenden pharmazeutisch gebräuchlichen Öls, welches ein
Triglycerid als wesentliche lipophile Komponente enthält; und c) 10-50 Gew.-%, bezogen auf die Trägerzusammensetzung, eines im wesentlichen reinen, oder als Gemisch vorliegenden nicht-ionischen Tensids mit einem HLB-Wert grösser als 10;
und gegebenenfalls weiteren pharmazeutisch annehmbaren Hilfsstoffen.

Ausserdem ist das Verfahren zur Herstellung einer pharmazeutischen Zusammensetzung mit einem solubilisierten, in Wasser schwerlöslichen Wirkstoff in einer Trägerzusammensetzung mit den genannten Komponenten Gegenstand der Erfindung. Diese pharmazeutische Zusammensetzung ist zur Abfüllung in oralen Dosiseinheitsformen, z.B. in Stärke-, Hartgelatine- oder Weichgelatinekapseln, geeignet.

Die weiter vom und im folgenden verwendeten Begriffe sind im Rahmen der Beschreibung der vorliegenden Erfindung wie folgt definiert:

Der Begriff pharmazeutische Zusammensetzung definiert das Gemisch eines solubilisierten, in Wasser schwerlöslichen pharmazeutischen Wirkstoffs oder Wirkstoffgemisches in einer Trägerzusammensetzung mit den genannten Komponenten, welches sich zu oralen Darreichungsformen, vorzugsweise Stärke-, Hartgelatine- oder Weichgelatinekapseln, verarbeiten lässt.

Der Begriff solubilisiert bzw. Solubilisierung eines in Wasser schwerlöslichen Wirkstoffs oder Wirkstoffgemisches definiert einen Dispersionsvorgang durch Einwirken eines geeigneten Löslichkeitsvermittlers, indem dieser die Dispersionsfähigkeit des Wirkstoffs so weit erhöht, dass eine therapeutisch wirksame Dosis vollständig gelöst oder zumindest durch einen partiellen Lösungsvorgang bioverfügbar wird. Der Begriff Dispersionsfähigkeit definiert ein Mass für die Bildung von Mikroemulsionen, echten molekularen Lösungen der Wirkstoffe und der Hilfsstoffe in Wasser sowie von kolloidalen Lösungen, z.B. Lösungen aus Assoziationskolloiden oder Molekülkolloiden, die klar bzw. opaleszierend sind, und gegebenenfalls nach Filtrieren, insbesondere mit sterilen Filtern mit 5-10 µm Porendurchmesser, keinerlei Feststoffpartikel aufweisen, oder z.B. mizellare Lösungen oder Sphärokolloide, die nur in der Ultrazentrifuge abtrennbar sind. Die Dispersionsfähigkeit kann z.B. in mg oder mMol pro Liter Wasser angegeben werden.

Ein in Wasser schwerlöslicher pharmazeutischer Wirkstoff oder Wirkstoffgemisch besitzt eine Löslichkeit in Wasser kleiner als 500 mg/1000 ml, vorzugsweise kleiner als 200mg/ml.

Besonders geeignete schwerlösliche Wirkstoffe sind Immunsuppressiva mit Makrolidstruktur, z.B. Cyclosporin A, Cyclosprin G, Rapamycin, Tacrolimus, Deoxyspergualin, Mycophenolate-Mofetil, Gusperimus, nicht-steroidale Antiphlogistika, z.B. Acetylsalicylsäure, Ibuprofen bzw. S(+)-Ibuprofen, Indomethacin, Diclofenac, Piroxicam, Meloxicam, Tenoxicam, Naproxen, Ketoprofen, Flurbiprofen, Fenoprofen, Felbinac, Sulindac, Etodolac, Oxyphenbutazon, Phenylbutazon, Nabumeton; Dihydropyridinderivate mit kardiovaskulärer Wirkung, z.B. Nifedipin, Nitrendipin, Nimodipin, Nisoldipin, Isradipin, Felodipin, Amlodipin, Nilvadipin, Lacidipin, Benidipin, Masnidipin, Fumidipin, Niguldipin, Neuraltherapeutika, z.B. α-Liponsäure, Muramylpeptide, z.B. Muramyldipeptid oder -tripeptid, Romurtid, fettlösliche Vitamine, z.B. Vitamin A, D, E oder F; Alkaloide, z.B. Vincopectin, Vincristin, Vinblastin, Reserpin, Codein, Mutterkorn-Alkaloide, z.B. Bromocriptin, Dihydroergotamin, Dihydroergocristin; Antitumormittel, z.B. Chlorambucil, Etoposid, Teniposid, Idoxifen, Tallimustin, Teloxantron, Tirapazamin, Carzelesin, Dexniguldipin, Intoplicin, Idarubicin, Miltefosin, Trofosfamid, Teloxantrone, Melphalan, Lomustin, 4,5-Bis(4'fluoranilino)-phthalimid; 4,5-Dianilinophthalimid.; Immunmodulatoren, z.B. Thymoctonan, Prezatid-Kupferacetat; Antiinfektiva, z.B. Erythromycin, Daunorubicin, Gramicidin, Doxorubicin, Amphotericin B, Gentamycin, Leucomycin, Streptomycin, Ganefromycin, Rifamexil, Ramoplanin, Spiramycin; Antimykotika, z.B. Fluconazol, Ketoconazol, Itraconazol; H2-Rezeptorantagonisten, z.B. Famotidin, Cimetidin, Ranitidin, Roxatidin, Nizatidin, Omeprazol, Proteinkinase-Hemmer, z.B. N-[4-Methyl-3-(4-pyridin-3-ylpyrimidin-2-ylamino)-phenyl]-benzamid, N-Benzyol-staurosporin; HIV-1-Protease-Inhibitoren, z.B. BOC-Phe^{c}Phe-Val-Phe-Morpholin oder sein O-[2-(2-methoxyethoxy)-acetoxy]-Derivat; Leukotrien-Antagonisten, z.B. N-[4-(5-Cyclopentyloxycarbonylamino-1-methylindol-3-ylmethyl)-3-methoxybenzoyl]-2-vinyloxy]-benzolsulfonamid.

Besonders bevorzugt sind Cyclosporine, Rapamycin, Tacrolimus, Deoxyspergualin, Mycophenolat-Mofetil, Nifedipin, Nimodipin, Etoposid, Ibuprofen und α-Liponsäure.

Statt des als freie Säure oder in basischer Form vorliegenden Wirkstoffs selbst kann in der pharmazeutischen Zusammensetzung der Wirkstoff in Form eines pharmazeutisch annehmbaren Salzes vorhanden sein, z.B. als Hydrobromid, Hydrochlorid, Mesylat, Acetat, Succinat, Lactat, Tartrat, Fumarat, Sulfat, Maleat, etc..

Die Konzentration des Wirkstoffs oder der Wirkstoffkombination ist durch die zu applizierende Dosis festgelegt. Sie kann 1 bis 30 Gew.-%, vorzugsweise 5 bis 20 Gew.-%, insbesondere 5 bis 12 Gew.-%, betragen, bezogen auf das Gewicht der Trägerzusammensetzung.

Die Trägerzusammensetzung für einen der genannten Wirkstoffe oder für eine Wirkstoffkombination ist wie folgt definiert:

Die Forderung "im wesentlichen rein" mit Bezug auf eine in der Trägerzusammensetzung vorhandene Komponente definiert einen Reinheitsgrad höher als 90 %, vorzugsweise höher als 95 %, dieser Komponente vor ihrem Vermischen mit den anderen Komponenten der Trägerzusammensetzung. Vorzugsweise hat eine als "im wesentlichen rein" definierte Komponente eine einheitlich definierte Struktur und Zusammensetzung.

Als Gemisch vorliegende Komponenten in der Trägerzusammensetzung können Gemische von Naturstoffen sein, deren Zusammensetzung vom Rohstoff selbst, seiner Isolierung und seiner Weiterverarbeitung bedingt ist. Die Bestandteile solcher Gemische sind in den Spezifikationen des Herstellers angegeben.

Der Polyglycerinfettsäureester der Komponente a) besteht aus einem im wesentlichen reinen oder dem Gemisch verschiedener Polyglycerinfettsäureester, worin der Polyglyceringrundkörper vorzugsweise bis einschliesslich 10 Glycerineinheiten enthält, welche mit 1-10 Säureresten von gesättigten oder ungesättigten Carbonsäuren und gerader Anzahl von 8-20 C-Atomen verestert sind.

Der Säurerest einer gesättigten Carbonsäure mit gerader Anzahl von 8-20 C-Atomen, welcher den Polyglyceringrundkörper verestert, ist vorzugsweise geradkettig mit 12, 14, 16 und 18 C-Atomen, z.B. n-Dodecanoyl, n-Tetradecanoyl, n-Hexadecanoyl oder n-Octadecanoyl.

Der Säurerest einer ungesättigten Carbonsäure mit gerader Anzahl von 8-20 C-Atomen, welcher den Polyglyceringrundkörper verestert, ist vorzugsweise geradkettig mit 12, 14, 16 und 18 C-Atomen und weist 1 Doppelbindung auf, z.B. 9-cis-Dodecenoyl, 9-cis-Tetradecenoyl, 9-cis-Hexadecenoyl oder 9-cis-Octadecenoyl.

Für die genannten Säurereste sind auch die in Klammern angegebenen Bezeichnungen gebräuchlich:

Für die genannten Säurereste sind ausserdem folgende Bezeichnungen gebräuchlich: 9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaldoyl), 11-cis-Octadecenoyl (Vaccenoyl), 9-cis-Icosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl).

Geeignete Polyglycerinfettsäureester mit einheitlich definierter Struktur sind beispielsweise Diglyceryl-monocaprate, Diglycerol-monolaurate, Diglycerol-diisostearate, Diglycerol-monoisostearate, Diglycerol-tetrastearate (Polyglyceryl-2-tetrastearate), Triglycerol-monooleate (Polyglyceryl-3-monooleate), Triglyceryl-monolaurate, Triglyceryl-monostearate (Polyglyceryl-3-stearate), Triglyceryl-monoisosterate, Hexaglyceryl-dioleate (Polyglycerol-6-dioleate), Hexaglyceryl-distearate (Polyglycerol-6-distearate), Decaglyceryl-dioleate (Polyglyceryl-10-dioleate), Diglycerol-tetraoleate (Polyglyceryl-10-tetraoleate), Decaglyceryl-decaoleate (Polyglyceryl-10-decaoleate), Decaglyceryl-decastearate (Polyglyceryl-10-decastearate). In Klammern ist die CTFA-Nomenklatur angegeben. Diese Produkte sind kommerziell unter den Wortzeichen Caprol® Warenzeichen der Fa.Karlshamns USA Inc., Columbus Ohio) erhältlich. Exakte Produktbezeichnungen: CAPROL 2G4S, 3GO, 3GS, 6G2O, 6G2S, 10G2O, 10G4O, 10G10O,10G10S. Weitere Produkte sind unter den Bezeichungen DGLC-MC, DGLC-ML, DGLC-DISOS, DGLC-MISOS, TGLC-ML und TGLC-MISOS bei der Fa. Solvay Alkali GmbH, D-3002 Hannover erhältlich.

Das Gemisch verschiedener Polyglycerinfettsäureester ist unter Bezeichnungen wie decaglycerol mono-, di-oleate, polyglycerol ester of mixed fatty acids, Polyglycerolester der Fettsäuren, polyglycerol caprate, cocoate, laurate, lanolinate, isostearate oder rizinolate definiert und kommerziell unter den Wortzeichen Triodan® und Homodan®(Warenzeichen der Fa. Grindsted Products, Grindsted Dänemark), exakte Produktbezeichnungen: TRIODAN 20, 55, R90 und HOMODAN MO, Radiamuls® (Warenzeichen der Fa. Petrofina (FINA), Bruxelles Belgien), exakte Produktbezeichnung RADIAMULS Poly 2253, der Bezeichnung CAPROL PGE 860 oder ET, oder den Wortzeichen Plurol®(Warenzeichen Gattefosé Etablissements, Saint-Priest, Frankreich), exakte Produktbezeichnung PLUROL Stearique WL 1009 oder PLUROL Oleique WL 1173, erhältlich. Weitere Produkte sind unter den Bezeichungen PGLC-C 1010 S, PGLC-C 0810, PGLC-C 1010/S, PGLC-L T 2010, PGLC-LAN 0510/S, PGLC-CT 2010/90, PGLC-ISOS T UE, PGLC-R UE, PGLC-ISOS 0410 bei der Fa. Solvay Alkali GmbH, D-3002 Hannover erhältlich.

Die genannten Polyglycerinfettsäureester erfüllen die im Foodchemical Codex FCC III unter "Monographs" auf S.232 genannten Angaben hinsichtlich "Description", "Requirements" und "Tests". Es gelten insbesondere die von den genannten Herstellern publizierten Produktbeschreibungen mit den Angaben auf Datenblättern für das betreffende Produkt, insbesondere Spezifikationen wie Gehalt an Monoester, Tropfpunkt, Freies Glycerol, Freie Fettsäure, Jodzahl, Form, Antioxidantien, HLB-Wert, Eigenschaften und Haltbarkeit.

Die genannten Polyglycerinfettsäureester erfüllen insbesondere die Anforderungen gemäss Nummer E 475 der EG-Lebensmittelzusatzstoffverordnung (EG-Richtlinie 74/329) sowie der Verordnung der U.S. FDA Code 21 CFR §172.854.

Der Sorbitanfettsäureester der Komponente a) besteht vorzugsweise aus einem im wesentlichen reinen oder dem Gemisch verschiedener Sorbitanfettsäureester, worin der Sorbitangrundkörper mit 1-3 Säureresten einer gesättigten oder ungesättigten, geradkettigen Carbonsäure und gerader Anzahl von 8-20 C-Atomen verestert ist.

Der Säurerest einer gesättigten Carbonsäure mit gerader Anzahl von 8-20 C-Atomen, welcher den Sorbitangrundkörper verestert, ist vorzugsweise geradkettig mit 12, 14, 16 und 18 C-Atomen, z.B. n-Dodecanoyl, n-Tetradecanoyl, n-Hexadecanoyl oder n-Octadecanoyl.

Der Säurerest einer ungesättigten Carbonsäure mit gerader Anzahl von 8-20 C-Atomen ist vorzugsweise geradkettig mit 12, 14, 16 und 18 C-Atomen, z.B Oleoyl.

Geeignete Sorbitanfettsäureester sind insbesondere Sorbitan-Monolaurat, -Monopalmitat, -Monostearat, -Tristearat, -Monooleat, -Sesquioleat und -Trioleat. Diese Produkte sind kommerziell unter den Wortzeichen Span® (Warenzeichen der Fa.Atlas, Wilmington USA), exakte Produktbezeichnungen: SPAN 20, 40, 60, 65, 80 und 85, Arlacel® (Warenzeichen der Fa.Atlas), exakte Produktbezeichnungen: ARLACEL 20, 40, 60, 80, 83, 85 und C, Crill® (Warenzeichen der Fa. Croda Chemicals Ltd., Cowick Hall, Snaith Goole GB), exakte Produktbezeichnungen: CRILL 1, 3 und 4, Dehymuls® (Warenzeichen der Fa. Henkel, Düsseldorf DE), exakte Produktbezeichnungen: DEHYMULS SML, SMO, SMS, SSO, Famodan® (Warenzeichen der Fa. Grindsted Products, Grindsted Dänemark), exakte Produktbezeichnungen: FAMODAN MS und TS, Capmul® (Warenzeichen der Fa.Karlshamns USA Inc., Columbus Ohio), exakte Produktbezeichnungen: CAPMUL S und O, Radiasurf® (Warenzeichen der Fa.Petrofina (FINA), Bruxelles Belgien), exakte Produktbezeichnungen: RADIASURF 7125, 7135, 7145 und 7155, erhältlich.

Die genannten Sorbitanfettsäureester und die genannten Polyglycerinfettsäureester erfüllen die in der Britischen Pharmakopöe (spezielle Monographie) oder Ph.Helv.VI genannten Angaben. Es gelten insbesondere die von den genannten Herstellern publizierten Produktbeschreibungen mit den Angaben auf Datenblättern für das betreffende Produkt, insbesondere Spezifikationen wie Form, Farbe, HLB-Wert, Viskosität, Steigschmelzpunkt und Löslichkeit.

Die Komponente a) besitzt einen HLB-Wert kleiner als 10. Sie ist in der Trägerzusammensetzung in einem Mengenanteil von 10-50 Gew.-%, bevorzugt 15-40 Gew.-%, vorzugsweise 15-20 Gew.-%, bezogen auf das Gesamtgewicht der Trägerzusammensetzung, vorhanden. Die Komponente a) kann auch aus Produktgemischen der genannten Polyglycerinfettsäureester untereinander oder Produktgemischen der genannten Sorbitanfettsäureester untereinander oder Produktgemischen der genannten Polyglycerinfettsäureester mit den genannten Sorbitanfettsäureestern bestehen.

Ein pharmazeutisch gebräuchliches Öl b) ist ein Triglycerid natürlichen Ursprungs oder ein synthetisches oder halbsynthetisches, im wesentlichen reines Triglycerid. Bevorzugt ist ein Triglycerid natürlichen Ursprungs, worin das Glycerin durch Säurereste von gesättigten oder ungesättigten Carbonsäuren mit gerader Anzahl von 8-20 C-Atomen verestert ist. Solche Säurereste sind weiter vom definiert, z.B. n-Dodecanoyl, n-Tetradecanoyl, n-Hexadecanoyl, n-Octadecanoyl oder Oleoyl.

Geeignete Triglyceride natürlichen Ursprungs sind z.B. Erdnuss-, Sesam-, Sonnenblumen-, Oliven-, Malskeim-, Soja-, Rizinus-, Baumwollsamen-, Raps-, Distel-, Traubenkern-, Fisch- oder Neutralöl.

Die Komponente b) ist in der Trägerzusammensetzung in einem Mengenanteil von 5-40 Gew.-%, vorzugsweise 10-35 Gew.-%, bezogen auf das Gesamtgewicht der Trägerzusammensetzung, vorhanden. Die Komponente b) kann auch aus Produktgemischen der genannten pharmazeutisch gebräuchlichen Öle bestehen.

Das nicht-ionische Tensid der Komponente c) mit einem HLB-Wert grösser als 10 ist vorzugsweise ein amphiphiler Stoff, dessen hydrophiler Bestandteil aus Polyethylenoxid besteht, wobei das mittlere Molekulargewichts des Polyethylenoxidanteils ca. 600-2500 beträgt, entsprechend 15-60 Ethylenoxideinheiten.

Geeignete nicht-ionische Tenside sind beispielsweise Reaktionsprodukte von natürlichem oder hydriertem Rizinusöl und Ethylenoxid. Solche Produkte sind z.B. kommerziell unter den Warenzeichen Cremaphor®, Niccol® und Emulgin® erhältlich. Geeignete nicht-ionische Tenside sind ebenfalls Polyoxyethylensorbitanfettsäureester (Polysorbate), z.B. POE-(20)-sorbitanmonolaurat, POE-(20)-sorbitanmonopalmitat, POE-(20)-sorbitantristearat, POE-(20)-sorbitanmonooleat oder POE-(20)-sorbitantrioleat sowie Polyoxyethylenfettsäureester, z.B. POE-(20, 30, 40, 50)-stearat. Solche Produkte sind z.B. kommerziell unter den Warenzeichen Tween® und Myrj® erhältlich.

Die Komponente c) ist in der Trägerzusammensetzung in einem Mengenanteil von 10-50 Gew.-%, vorzugsweise 20-45 Gew.-%, bezogen auf das Gesamtgewicht der Trägerzusammensetzung, vorhanden. Die Komponente c) kann auch aus Produktgemischen der genannten pharmazeutisch gebräuchlichen, nicht-ionischen Tenside bestehen.

Geeignete pharmazeutisch annehmbare, zusätzliche Hilfsstoffe werden der Trägerzusammensetzung in der Menge zugesetzt, dass sie sich mit den Mengen der Komponenten a), b) und c) sowie dem Wirkstoff oder der Wirkstoffkombination zu 100 Gew.-% ergänzen. Zusätzliche Hilfsstoffe können in Mengen von 0 % bis 75 Gew.-% in der Trägerzusammensetzung vorhanden sein. Zusätzliche Hilfsstoffe sind durch die Wahl der pharmazeutischen Darreichungsform bedingt. Für flüssige Darreichungsformen wie Tropfen, Suspensionen oder Kapselfüllungen setzt man pharmazeutisch zulässige, übliche Verdünnungsmittel hinzu, wie Ethanol, Propanol, Isopropanol, Propylenglycol, Polyethylenglycol, Glycerin oder Wasser oder Gemische davon.

Ferner kann man übliche Hilfsstoffe hinzusetzen, z.B. Konservierungsmittel, z.B. Benzylalkohol, Ethanol, p-Hydroxybenzoesäureester, Sorbinsäure, Antioxidantien, z.B. Tocopherole, Butylhydroxyanisol, Butylhydroxytoluol, Ascorbinsäure, Ascorbylpalmitat; Stabilisatoren, z.B. Citronensäure, Weinsäure, EDTA, Geschmacksstoffe oder Aromastoffe.

Für Kapselfüliungen von Gelatinekapseln eignen sich übliche Konsistenzmittel oder Weichmacher zum Erhalt einer stabilen Gelatinehülle. Solche Hilfsstoffe sind z.B. Sorbit, Sorbitan, Polyvinylpyrrolidon, Hydroxypropylmethylcellulose (HPMC), Hydroxypropylcellulose, Methylcellulose oder kolloidales Siliciumdioxid.

Ebenfalls Gegenstand der Erfindung ist das Verfahren zur Herstellung der weiter vorn definierten pharmazeutischen Zusammensetzung, welches dadurch gekennzeichnet ist, dass man die Komponenten a), b) und c) und gegebenenfalls weitere pharmazeutisch annehmbare Hilfsstoffe in beliebiger Reihenfolge miteinander vermischt, in diesem Gemisch den in Wasser schwerlöslichen pharmazeutischen Wirkstoff dispergiert und gewünschtenfalls die Dispersion in eine geeignete, oral verabreichbare Form bringt.

Das Dispergieren des Wirkstoffs oder der Wirkstoffkombination kann nach dem Vermischen der Komponenten a), b) und c) sowie der übrigen Hilfsstoffe erfolgen. Alternativ kann man den Wirkstoff oder die Wirkstoffkombination in einer einzelnen Komponente oder einem Gemisch von zwei der genannten Komponenten dispergieren und die restlichen Komponenten hinzufügen. Solubilisierungs- bzw. Dispersionsvorgänge kann man durch Erwärmen von Einzelkomponenten oder Mischungen davon beschleunigen. Bevorzugt sind Reaktionsbedingungen, welche die Bildung einer kolloid dispersen Phase begünstigen.

In Gegenwart von sauerstoffempfindlichen Wirkstoffen führt man das Verfahren unter Schutzgasatmosphäre durch, z.B. unter Stickstoff, Helium oder Argon. Zuvor kann man in den flüssigen Komponenten vorhandenen Sauerstoff durch Anlegen von Unterdruck, z.B. 50-100 mbar, oder mittels Behandlung mit Ultraschall, entfernen. Für das genannte Verfahren eignet sich ein Reaktionsbehälter mit Doppelwand und Rührwerk.

Die Überführung in eine oral verabreichbare Darreichungsform erfolgt in an sich bekannter Weise. Zur Herstellung von oralen flüssigen Darreichungsformen wie Tropfen, Suspensionen, Emulsionen etc. bedient man sich der üblichen in Standardwerken wie Hagers Handbuch der Pharmazeutischen Praxis oder Remington's Pharmaceutical Sciences angegebenen Methodik.

Kapseln sind vorzugsweise Steckkapseln aus Gelatine, welche gegebenenfalls unter Zusatz von Glycerin oder Sorbit hergestellt werden, und sich durch Einwirken von Magensaft ohne zeitliche Verzögerung auflösen. Alternativ sind Kapseln aus Stärke verwendbar, z.B. die unter dem Warenzeichen Capill® der Fa. Capsugel/Warner Lambert erhältliche Handelsware. Kapseln können weitere Hilfs- und Füllstoffe wie Lactose, Stärke, Gleitmittel, wie Stärke oder Magnesiumstearat, beigemischt sein. In weichen Kapseln können zusätzlich Flüssigkeiten wie Lecithin, Fette, Öle, Parafinöl oder flüssiges Polyethylenglycol enthalten sein. Geeignet sind, abhängig von der Dosierung, Steckkapseln der Grössen 0-4, vorzugsweise 0-2. Es eignet sich die Handelsware der Firmen Shionogi, Capsugel oder Scherer.

Die folgenden Beispiele illustrieren die Erfindung ohne ihren weiter vorn definierten allgemeinen Umfang zu beschränken. Die genannten Wirkstoffe sind repräsentativ für sämtliche weiter vorn genannten Wirkstoffe. Temperaturangaben in Grad Celsius.

### Beispiel 1

### Rezeptur zur Abfüllung in Weichgelatinekapseln; Mengenangaben in mg pro fertiger Kapsel, Weichgelatinekapselformat: 22 minims oblong.

| | | |
|---|---|---|
| 1 | Ciclosporin A (USP XXII/Pharm.Eur.) | 100,0 |
| 2 | POE-(40)-hydriertes Rizinusöl (CREMOPHOR RH 40, NICCOL HCO 40, SIMULSOL 1293) | 400,0 |
| 3 | Di/tri/tetraglycerolfettsäureester (FCC/ TRIODAN 20) | 238,0 |
| 4 | Sesamöl (DAB 10) | 160,0 |
| 5 | alpha-Tocopherol (DAB 10) | 2,0 |
| 6 | Ethanol (DAB 10) | 100,0 |

Die Bestandteile 2-4 werden unter Erwärmen auf 40° in einem Edelstahlkessel mit Rührer vermischt. Danach wird die Lösung durch Anlegen von Unterdruck entgast. Zur klaren Lösung fügt man das Antioxidans 5 hinzu und dispergiert danach darin den Wirkstoff Ciclosporin A. Nach Zugabe des Ethanols rührt man den gesamten Ansatz bis zum Erhalt einer klaren Lösung. Nach Abkühlen auf ca.20° füllt man diese in Weichgelatinekapseln. Zum Ausgleich von Verdunstung enthält der Ansatz gegenüber obiger Rezeptur eine um 30-60 mg erhöhte Menge Ethanol.

Die Wandungen der Weichgelatinekapseln enthalten zusätzlich zur Gelatine Hilfsstoffe, welche die Konsitenz beeinflussen, z.B. Glycerin und/oder Propylenglycol oder Sorbit und/oder Mannit. Die Wandungen können zusätzlich Pigmente oder Farbstoffe enthalten, z.B. Titandioxid, Eisenoxid, Chinolingelb, oder Cochenillerot A.

### Beispiel 2

### Rezeptur zur Abfüllung in Hartgelatinekapseln oder Stärkekapseln; Mengenangaben in kg pro Ansatz.

| | | |
|---|---|---|
| 1 | Nifedipin (DAB 10) | 20,0 |
| 2 | POE-(20)-sorbitanmonooleat (Polysorbat 20 Pharm.Eur., TWEEN 20) | 168,0 |
| 3 | Triglycerol-mono/dioleat (FCC - CAPROL 3GO) | 28,0 |
| 4 | Neutralöl (MIGLYOL 812, CAPTEX 300/400) | 84,0 |

Alle Bestandteile der Rezeptur werden in einem doppelwandigen Heizkessel mit 300 l Fassungsvolumen bei 45° vermischt und bis zum Erhalt einer klaren Lösung gerührt. Jeweils 300 mg der klaren, abgekühlten Lösung werden in Hartgelatinekapseln der Grösse 1 abgefüllt, welche mit Titandioxid/Eisenoxid opakisiert sind.

Die gefüllten Kapseln werden mit einer Banderolle versehen. Wegen der Lichtempfindlichkeit des Nifedipins sind sämtliche Verarbeitungsschritte unter Ausschluss von Tageslicht durchzuführen.

### Beispiel 3

### Rezeptur zur Abfüllung in Glasflasche. Die Formulierung eignet sich zur oralen Verabreichung als Tropflösung, welche in einer braunen 40 ml Tropfflasche abgefüllt wird. Mengenangaben in Gramm.

| | | |
|---|---|---|
| 1 | Nimodipin | 3,0 |
| 2 | POE-(60)-hydriertes Rizinusöl (CREMOPHOR RH 60, NICCOL HCO 60, SIMULSOL 1294) | 15,0 |
| 3 | Sorbitanmonolaurat (BPC 1973, SPAN 20) | 8,5 |
| 4 | Sonnenblumenöl (DAB 10) | 8,5 |
| 5 | Propylenglycol | 5,0 |

Die Herstellung der Lösung erfolgt analog Beispiel 2.

### Beispiel 4

### Rezeptur zur Abfüllung in Weichgelatinekapseln; Mengenangaben in mg pro fertiger Kapsel, Weichgelatinekapselformat: 4 minims oblong.

| | | |
|---|---|---|
| 1 | Tacrolimus | 10,0 |
| 2 | POE-(35)-Rizinusöl (CREMOPHOR EL) | 72,0 |
| 3 | Sorbitanmonooleat (SPAN 80) | 72,0 |
| 4 | Neutralöl | 32,0 |
| 5 | alpha-Tocopherol | 1,0 |
| 6 | Propylenglycol (DAB 10) | 5,0 |

Die Herstellung der Kapseln erfolgt analog Beispiel 1. Als Konsistenzgeber für die Kapselwand ist besonders Propylenglycol geeignet.

### Beispiel 5

### Rezeptur zur Abfüllung in Hartgelatinekapseln; Mengenangaben beziehen sich auf die Füllung einer Kapsel der Grösse O.

| | | |
|---|---|---|
| 1 | alpha-Liponsäure | 100,0 |
| 2 | POE-(40)-stearat (US/NF, MYRJ 52 S) | 80,0 |
| 3 | Tetraglycolstearat (FCC, TRIODAN 55) | 215,0 |
| 4 | Sesamöl | 160,0 |
| 5 | Butylhydroxyanisol | 0,5 |

Die Herstellung der Lösung erfolgt analog Beispiel 2. Dabei ist zusätzlich die Sauerstoff-Empfindlichkeit der Liponsäure zu beachten.

### Beispiel 6

### Rezeptur zur Abfüllung in Weichgelatinekapseln; Mengenangaben in mg pro fertiger Kapsel, Weichgelatinekapselformat: 6 minims, oblong.

| | | |
|---|---|---|
| 1 | Rapamycin | 20,0 |
| 2 | POLYSORBAT 80 (TWEEN 80) | 150,0 |
| 3 | Sorbitanmonoleat | 25,0 |
| 4 | Neutralöl | 75,0 |
| 5 | Ascorbylpalmitat | 0,5 |
| 6 | Benzylalkohol (DAB 10) | 5,0 |

Die Herstellung erfolgt analog Beispiel 1, wobei der Benzylalkohol als letzter Bestandteil hinzugefügt wird.

### Beispiel 7

### Rezeptur zur Abfüllung in Weichgelatinekapseln; Mengenangaben in mg pro fertiger Kapsel.

| | | |
|---|---|---|
| 1 | Etoposid | 100,0 |
| 2 | POE-(40)-hydriertes Rizinusöl | 400,0 |
| 3 | Di-/tri-/tetraglycerollaurat (TGLC-Laurat T2010 Solvay Alkali GmbH) | 160,0 |
| 4 | Maisöl | 230,0 |
| 5 | Ethanol | 100,0 |

Die Herstellung erfolgt analog Beispiel 1.

### Beispiel 8

### Rezeptur zur Abfüllung in Weichgelatinekapseln; Mengenangaben in mg pro fertiger Kapsel, Weichgelatinekapselformat: 9,5 minims, oblong.

| | | |
|---|---|---|
| 1 | S(+)-Ibuprofen | 100,0 |
| 2 | POLYSORBAT 60 (TWEEN 60) | 210,0 |
| 3 | Hexaglyceroldioleat (CAPROL 6G2O) | 130,0 |
| 4 | Rizinusöl (DAB 10) | 60,0 |

Die Herstellung erfolgt analog Beispiel 1.

## Patentansprüche

1. Pharmazeutische Zusammensetzung zur Solubilisierung eines in Wasser schwerlöslichen Wirkstoffs, wobei Cyclosporine ausgenommen sind, in einer Trägerzusammensetzung, dadurch gekennzeichnet, dass die Trägerzusammensetzung die Komponenten:
a) 10-50 Gew.-%, bezogen auf die Trägerzusammensetzung, eines im wesentlichen reinen, oder als Gemisch vorliegenden Co-Tensids, mit einem Hydrophil-Lipophil-Gleichgewicht kleiner als 10 (HLB-Wert nach Griffin), ausgewählt aus der Gruppe Polyglycerinfettsäureester und Sorbitanfettsäureester;
b) 5-40 Gew.-%, bezogen auf die Trägerzusammensetzung, eines im wesentlichen reinen, oder als Gemisch vorliegenden pharmazeutisch gebräuchlichen Öls, welches ein Triglycerid als wesentliche lipophile Komponente enthält; und
c) 10-50 Gew.-%, bezogen auf die Trägerzusammensetzung, eines im wesentlichen reinen, oder als Gemisch vorliegenden nicht-ionischen Tensids mit einem HLB-Wert grösser als 10;
und gegebenenfalls weitere pharmazeutisch annehmbare Hilfsstoffe enthält.

2. Pharmazeutische Zusammensetzung nach Anspruch 1 zur Solubilisierung von 1-30 Gew.-%, bezogen auf das Gesamtgewicht der Trägerzusammensetzung, eines schwerlöslichen Wirkstoffs mit einer Löslichkeit kleiner als 500 mg/1000 ml in reinem Wasser.

3. Pharmazeutische Zusammensetzung nach Anspruch 1 oder 2 zur Solubilisierung eines schwerlöslichen Wirkstoffs ausgewählt aus der Gruppe Rapamycin, Tacrolimus, Deoxyspergualin, Mycophenolat-Mofetil, Nifedipin, Nimodipin, Etoposid, und Ibuprofen.

4. Pharmazeutische Zusammensetzung nach einem der Ansprüche 1 - 3, dadurch gekennzeichnet, dass die Komponente a) aus einem im wesentlichen reinen oder dem Gemisch eines Polyglycerinfettsäureesters besteht, worin der Polyglyceringrundkörper bis einschliesslich 10 Glycerineinheiten enthält, welche mit 1-10 Säureresten von gesättigten oder ungesättigten Carbonsäuren und gerader Anzahl von 8-20 C-Atomen verestert sind.

5. Pharmazeutische Zusammensetzung nach Anspruch 4, dadurch gekennzeichnet, dass die Komponente a) als Polyglycerinfettsäureester im wesentlichen reines Polyglyceryl-2-Tetrastearat, -3-Monooleat, -3-Stearat, -6-Dioleat, -6-Distearat, -10-Dioleat, -10-Tetraoleat, -10-Decaoleat oder -10-Decastearat oder Gemische dieser Verbindungen enthält.

6. Pharmazeutische Zusammensetzung nach einem der Ansprüche 1 - 3, dadurch gekennzeichnet, dass die Komponente a) aus einem im wesentlichen reinen oder dem Gemisch eines Sorbitanfettsäureester besteht, worin der Sorbitangrundkörper mit 1-3 Säureresten von gesättigten oder ungesättigten Carbonsäuren und gerader Anzahl von 8-20 C-Atomen verestert ist.

7. Pharmazeutische Zusammensetzung nach Anspruch 6, dadurch gekennzeichnet, dass die Komponente a) als Sorbitanfettsäureester im wesentlichen reines Sorbitan-Monolaurat, -Monopalmitat, -Monostearat, -Tristearat, -Monooleat, -Sesquioleat oder -Trioleat oder Gemische dieser Verbindungen enthält.

8. Pharmazeutische Zusammensetzung nach einem der Ansprüche 1-7, dadurch gekennzeichnet, dass die Komponente b) als pharmazeutisch gebräuchliches Öl Erdnuss-, Sesam-, Sonnenblumen-, Oliven-, Maiskeim-, Soja-, Rizinus-, Baumwollsamen-, Raps-, Distel-, Traubenkern-, Fisch- oder Neutralöl und die Komponente c) ein nicht-ionisches Tensid mit einem hydrophilen Bestandteil bestehend aus 15-60 Ethylenoxideinheiten enthält.

9. Verfahren zur Herstellung einer pharmazeutischen Zusammensetzung gemäss Anspruch 1, dadurch gekennzeichnet, dass man die Komponenten a), b) und c) und gegebenenfalls weitere pharmazeutisch annehmbare, wasserlösliche Hilfsstoffe in beliebiger Reihenfolge miteinander vermischt, in diesem Gemisch den in Wasser schwerlöslichen pharmazeutischen Wirkstoff dispergiert und gewünschtenfalls die Dispersion in eine geeignete, oral verabreichbare Form bringt.

10. Verfahren gemäss Anspruch 9, dadurch gekennzeichnet, dass man die Dispersion in Stärke-, Härtgelatine- oder Weichgelatinekapseln abfüllt.

## Claims

1. A pharmaceutical composition for the solubilisation of an active ingredient that is sparingly soluble in water, excluding cyclosporins, in a carrier composition, wherein the carrier composition comprises the components:
a) from 10 to 50% by weight, based on the carrier composition, of a co-surfactant that is substantially pure or that is in the form of a mixture, having a hydrophilic-lipophilic balance of less than 10 (HLB value according to Griffin), selected from the group consisting of polyglycerol fatty acid esters and sorbitan fatty acid esters;
b) from 5 to 40% by weight, based on the carrier composition, of a pharmaceutically acceptable oil that is substantially pure or that is in the form of a mixture, comprising a triglyceride as main lipophilic component; and
c) from 10 to 50% by weight, based on the carrier composition, of a non-ionic surfactant that is substantially pure or that is in the form of a mixture, having a HLB value of more than 10;
and, optionally, further pharmaceutically acceptable excipients.

2. A pharmaceutical composition according to claim 1 for the solubilisation of from 1 to 30% by weight, based on the total weight of the carrier composition, of a sparingly soluble active ingredient having a solubility in pure water of less than 500 mg/1000 ml.

3. A pharmaceutical composition according to either claim 1 or claim 2 for the solubilisation of a sparingly soluble active ingredient selected from the group consisting of rapamycin, tacrolimus, deoxyspergualin, mycophenolate-mofetil, nifedipine, nimodipine, etoposide and ibuprofen.

4. A pharmaceutical composition according to any one of claims 1 to 3, wherein component a) consists of a substantially pure polyglycerol fatty acid ester or of a mixture of polyglycerol fatty acid esters, wherein the polyglycerol chain contains up to and including 10 units of glycerol that have been esterified with from 1 to 10 acid radicals of saturated or unsaturated carboxylic acids having an even number of from 8 to 20 carbon atoms.

5. A pharmaceutical composition according to claim 4, wherein component a) comprises as polyglycerol fatty acid ester substantially pure polyglyceryl 2-tetrastearate, polyglyceryl 3-monooleate, polyglyceryl 3-stearate, polyglyceryl 6-dioleate, polyglyceryl 6-distearate, polyglyceryl 10-dioleate, polyglyceryl 10-tetraoleate, polyglyceryl 10-decaoleate or polyglyceryl 10-decastearate or a mixture of those compounds.

6. A pharmaceutical composition according to any one of claims 1 to 3, wherein component a) consists of a substantially pure sorbitan fatty acid ester or a mixture of sorbitan fatty acid esters, wherein the sorbitan skeleton has been esterified with from 1 to 3 acid radicals of saturated or unsaturated carboxylic acids having an even number of from 8 to 20 carbon atoms.

7. A pharmaceutical composition according to claim 6, wherein component a) comprises as sorbitan fatty acid ester substantially pure sorbitan monolaurate, sorbitan monopalmitate, sorbitan monostearate, sorbitan tristearate, sorbitan monooleate, sorbitan sesquioleate or sorbitan trioleate or a mixture of those compounds.

8. A pharmaceutical composition according to any one of claims 1 to 7, wherein component b) comprises as pharmaceutically acceptable oil groundnut oil, sesame oil, sunflower oil, olive oil, corn oil, soybean oil, castor oil, cottonseed oil, rapeseed oil, thistle oil, grapeseed oil, fish oil or neutral oil, and component c) comprises a non-ionic surfactant having a hydrophilic component consisting of from 15 to 60 units of ethylene oxide.

9. A process for the preparation of a pharmaceutical composition according to claim 1, which comprises mixing components a), b) and c) with one another and, optionally, with further pharmaceutically acceptable water-soluble excipients, in any order, dispersing in that mixture a pharmaceutical active ingredient that is sparingly soluble in water and, if desired, processing the dispersion to a suitable dosage form for oral administration.

10. A process according to claim 9, which comprises introducing the dispersion into starch capsules or hard or soft gelatin capsules.

## Revendications

1. Une composition pharmaceutique pour la solubilisation d'une substance active difficilement soluble dans l'eau, les Cyclosporines étant exclues, dans une composition véhiculeuse, caractérisée en ce que la composition véhiculeuse comprend comme composants:
a) 10-50% en poids par rapport à la composition véhiculeuse d'un tensio-actif essentiellement pur ou présent sous forme d'un mélange, avec une balance hydrophilelipophile inférieure à 10 (valeur du H.L.B. selon Griffin), choisi parmi les esters de l'acide gras du polyglycérol et les esters de l'acide gras du sorbitane;
b) 5-40% en poids par rapport à la composition véhiculeuse d'une huile pharmaceutiquement acceptable, essentiellement pure ou présente sous forme d'un mélange qui contient un triglycéride comme composant lipophile essentiel; et
c) 10-50% en poids par rapport à la composition véhiculeuse d'un tensio-actif non ionique essentiellement pur ou présent sous forme d'un mélange ayant une valeur du H.L.B. supérieure à 10;
et, éventuellement d'autres excipients pharmaceutiquement acceptables.

2. Une composition pharmaceutique selon la revendication 1 pour la solubilisation de 1-30 % en poids par rapport au poids total de la composition véhiculeuse d'une substance active difficilement soluble ayant une solubilité inférieure à 500 mg/1000 ml dans de l'eau pure.

3. Une composition pharmaceutique selon la revendication 1 ou 2 pour la solubilisation d'une substance active difficilement soluble choisie parmi le groupe de la Rapamycine, du Tacrolimus, de la Désoxyspergualine, du Mycophénolate de Mofetil, de la Nifédipine, de la Nimodipine, de l'Etoposide et de l'Ibuprofène.

4. Une composition pharmaceutique selon l'une des revendications 1 à 3, caractérisée en ce que le composant a) est constitué d'un ester de l'acide gras du polyglycérol essentiellement pur ou d'un de ses mélanges où la chaîne principale du polyglycérol contient jusqu'à et y compris 10 unités de glycérol, qui sont estérifiées avec 1-10 restes acides d' acides carboxyliques saturés ou insaturés et ayant un nombre pair de 8-20 atomes de carbone.

5. Une composition pharmaceutique selon la revendication 4, caractérisée en ce que le composant a) contient comme ester de l'acide gras du polyglycérol, du 2-tétrastéarate de polyglycéryle, du 3-monooléate de polyglycéryle, du 3-stéarate de polyglycéryle, du 6-dioléate de polyglycéryle, du 6-distéarate de polyglycéryle, du 10-dioléate de polyglycéryle, du 10-tétraoléate de polyglycéryle, du 10-décaoléate de polyglycéryle ou du 10-décastéarate de polyglycéryle essentiellement purs ou des mélanges de ces composés.

6. Une composition pharmaceutique selon l'une des revendications 1 à 3, caractérisée en ce que le composant a) est constitué d'un ester de l'acide gras du sorbitane essentiellement pur ou d'un de ses mélanges où la chaîne principale du sorbitane est estérifiée avec 1-3 restes acides d'acides carboxyliques saturés ou insaturés et ayant un nombre pair de 8-20 atomes de carbone.

7. Une composition pharmaceutique selon la revendication 6, caractérisée en ce que le composant a) contient comme ester de l' acide gras du sorbitane du monolaurate de sorbitane, du monopalmitate de sorbitane, du monostéarate de sorbitane, du tristéarate de sorbitane, du monooléate de sorbitane, du sesquioléate de sorbitane ou du trioléate de sorbitane essentiellement purs ou des mélanges de ces composés.

8. Une composition pharmaceutique selon l'une des revendications 1 à 7, caractérisée en ce que le composant b) contient comme huile pharmaceutiquement acceptable, de l'huile d'arachides, de l'huile de sésame, de l'huile de tournesol, de l'huile d'olive, de l'huile de maïs, de l'huile de soja, de l'huile de ricin, de l'huile de coton, de l'huile de colza, de l'huile de chardon, de l'huile de pépins de raisins, de l'huile de poisson ou de l'huile neutre et le composant c) contient un tensio-actif non ionique ayant un composant hydrophile constitué de 15-60 unités d'oxyde d'éthylène.

9. Un procédé de préparation d'une composition pharmaceutique selon la revendication 1, caractérisé en ce qu'on mélange dans n'importe quel ordre les composants a), b) et c) et éventuellement d'autres excipients solubles dans l'eau et pharmaceutiquement acceptables, on disperse dans ce mélange la substance active pharmaceutique difficilement soluble dans l'eau et éventuellement, on amène la dispersion sous une forme appropriée pour une administration par voie orale.

10. Un procédé selon la revendication 9, caractérisé en ce que l'on introduit la dispersion dans des gélules d'amidon, des gélules de gélatine dures ou des gélules de gélatine molles.
